# EUROPEAN PATENT SPECIFICATION

(11) **EP 1 096 909 B1**
(45) Date of publication and mention of the grant of the patent: **09.04.2003**
(21) Application number: 99933445.1
(22) Date of filing: 06.07.1999
(51) Int. Cl.: A61F 13/15

(54) **SWELLABLE ABSORBENT PRODUCT AND A METHOD FOR ITS MANUFACTURE**
QUELLBARES SAUGFÄHIGES PRODUKT UND VERFAHREN ZU SEINER HERSTELLUNG
PRODUIT ABSORBANT GONFLANT ET SON PROCEDE DE FABRICATION

(30) Priority: 10.07.1998 SE 9802502
(43) Date of publication of application: 09.05.2001
(73) Proprietor: SCA Hygiene Products AB, 405 03 Göteborg (SE)
(72) Inventor: SAMUELSSON, Ann, S-437 32 Lindome (SE); PERSSON, Charlotte, S-412 57 Göteborg (SE); CABELDUC, Pascale, S-417 17 Göteborg (SE); DREVIK, Solgun, S-435 35 Mölnlycke (SE)
(74) Representative: Romare, Laila Anette
(86) International application number: SE9901225
(87) International publication number: WO00002509

(56) References cited:
- EP-A- 0 768 072
- EP-A1- 0 804 917
- EP-A1- 0 834 296
- WO-A1-98/08475
- US-A- 5 853 401

## Description

The invention relates to an absorbent product, such as a sanitary towel, an incontinence pad or a panty liner, which product has an essentially elongate shape with a longitudinal direction and a transverse direction, an upper side and a lower side, and has two end portions and a central portion located between the end portions.

The invention also relates to a method of producing an absorbent product.

### BACKGROUND

Conventional absorbent products of the type referred to above usually have a plane shape. Such a shape is suitable for transport and storage of the products because plane-shaped products occupy less space than non-planar products. During use, however, a plane-shaped product tends to follow the undergarments of the wearer rather than the body of the wearer, and the risk of leakage becomes imminent in the event of a gap being formed between the product and the wearer.

It has therefore been proposed that absorbent products be provided with a raised portion which fits better against the body of the wearer and results in the product maintaining contact with the body. Such solutions are described in, for example, PCT/SE97/01886. The raised portions can be non-absorbent so that the fluid discharged by the wearer is conveyed along or through the raised portion to absorbent parts of the product. It is more common, however, for the raised portion to contain absorbent material, so that the raised portion also can retain fluid. Such a construction makes the overall absorption capacity greater.

A problem with constructing raised portions from absorbent material is that the absorbent material swells in an uncontrolled manner and can lead to the raised portion assuming a shape which is undesirable from the point of view of comfort and function, or swells to such a height that it causes discomfort for the wearer.

An alternative, so as to avoid the raised portion swelling in an uncontrolled manner, is not to use hard-compressed material or a superabsorbent mixture. In many cases, however, such a method results in the overall absorption capacity being too low.

In EP-A-0 804 917 and EP-A- 0 834 296, absorbent articles are disclosed which are capable of self-shaping in use. The articles comprise absorbent material which will expand when activated by body fluids. In order to accomodate the expansion, excess or extensible topsheet may be provided.

### OBJECT OF THE INVENTION

An object of the invention is to remedy the problems referred to above and construct an absorbent product which swells in a controlled manner.

### BRIEF DESCRIPTION OF THE INVENTION

A product of the type referred to in the introduction, in which problems associated with previously known such products are essentially eliminated, is, according to the invention, characterized in that the product comprises means for controlled swelling of the raised portion on wetting, said means comprising fastening points made of water-soluble material between a liquid-permeable surface layer and the raised portion.

A product according to the invention can also be characterized in that the raised portion consists of a separate piece of material. Alternatively, the raised portion constitutes a part of the absorption body of the product.

According to one embodiment of the invention, the absorbent product is characterized in that a liquid-permeable surface layer is laid over the upper side of the entire product. According to another embodiment, the raised portion is surrounded by another surface layer.

According to the invention, the means for controlled swelling can comprise attachment points between the surface material and the raised portion. The means for controlled swelling can also comprise a stretchable surface material. Alternatively, the means for controlled swelling can comprise an excess of surface material.

If the product comprises a porous absorption material which is held down by attachment areas, the means for controlled swelling can comprise attachment points made of water-soluble material. The water-soluble material can be polyvinyl alcohol.

The invention also relates to a method of producing an absorbent structure which can constitute a raised portion in an absorbent product according to the invention. The method comprises the following steps:
a) a compressible strip of absorption material is laid down in such a shape that loops of excess material are formed in various areas of the strip,
b) the strip is compressed so that the loops are essentially obliterated and areas with a greater degree of compression alternating with areas with a smaller degree of compression are formed,
c) a long, narrow body is punched out of the compressed strip,
d) the body is arranged on a surface layer arranged in a mould with depressions,
e) the surface layer is attached discontinuously with a water-soluble material to the absorption body so that an excess of surface layer is obtained between the attachment areas.

### DESCRIPTION OF THE INVENTION

According to the invention, the abovementioned problems are solved by virtue of the fact that the raised portion is provided with means for controlled swelling.

Such a means can consist in that the absorbent material which is to constitute the raised portion is compressed partly within limited areas. A surface material is fastened over the raised portion and the raised portion is fastened on the absorbent product. The fastening of the surface material to the absorption material on the raised portion is effected in such a manner that controlled swelling of the absorption material is achieved. This can be effected by the surface material being attached in such a manner that parts of the absorption material and the surface material are left without attachment areas. When fluid is absorbed by the absorption material, this will be allowed to swell in a controlled manner. In this case, the absorption material can be, for example, hard-compressed cellulose pulp, hard-compressed cotton with a binding fibre mixed in, or regenerated cellulose such as rayon fibre. In order that the surface material can follow the swelling, it is necessary that the surface material can stretch or that excess surface material is located in suitable areas. The surface material can be nonwoven and the fastening areas can consist of, for example, hot-melt adhesive or welded joints. If the surface material is made of natural fibre, it is suitable to mix in binding fibres, so that the fastening points can be formed by melting these binding fibres by supplying heat and pressure. It is also possible to create fastening points by means of ultrasound. According to a preferred embodiment, the surface material consists of cellulose-based fibres such as, for example, rayon or cotton, with polyvinyl alcohol fibre mixed in.

The means for controlled swelling can also comprise a porous absorption material, the volume being kept down by the surface material being fastened down over the absorption material in the attachment areas. The surface material can be present in excess. The attachment areas consist of material which is dissolved on wetting, the absorption material then being able to swell as the surface material is released. The absorption material can consist of a foamed material, a wadding material, for example polyester wadding, or another compressible and resilient absorbent material. The attachment areas are suitably formed by water-soluble adhesive. Mixing a water-soluble fibre into the surface material, such as polyvinyl alcohol, which fibres are melted by supplying heat, is another method of forming fastening areas which dissolve on wetting.

The raised portion can constitute an integral part of the absorption body or consist of a separate piece of material. It is possible to have an entirely obliterated raised portion when the product is delivered, which swells to form a raised portion on wetting. This is effected by the material being compressed harder where the raised portion is to be formed. It is more advantageous, however, if the raised portion is already present on delivery to the wearer. It is easier for the wearer to position the product correctly against the body if a raised portion is already present. On wetting, the raised portion is then allowed to swell in a controlled manner to a height and shape which results in it performing its function and at the same time being comfortable for the wearer.

The raised portion is to lie in a centred position in the transverse direction on the product, that is to say equidistantly from the long sides of the product. The raised portion can be positioned roughly centrally on the product in the longitudinal direction also, that is to say equidistantly from the short sides of the product Alternatively, however, the raised portion can be displaced from the centre-in the longitudinal direction, depending on the appearance of the product in other respects. If the product has a front end portion intended to be positioned over the mons pubis of the wearer and a rear end portion intended to fit between the buttocks of the wearer with the rear short side located between the buttocks of the wearer, the raised portion is suitably displaced towards the back of the product. If, on the other hand, the rear end portion is lengthened so as to extend around the buttocks of the wearer and reach the place where the buttocks and the lower back meet the raised portion is suitably displaced towards the front of the product. This is often the case in the construction of what are known as night towels. When the wearer lies on her back, the lengthened rear end portion is to protect against leakage backwards. As described above, the raised portion is to be roughly centrally positioned. The crucial factor is that the raised portion is located in such a manner that, when the product is positioned correctly, it fits against the vestibule of the wearer so as to be capable of meeting the menstrual fluid as soon as it leaves the body, as a result of which the risk of leakage is reduced. It is often desirable, however, for the raised portion to be arranged equidistantly from the long sides of the product in the central portion of the product

The construction described for controlled swelling can of course be used in other places in the product than in a centrally positioned raised portion. For example, leakage barriers can be created along the long sides or short sides of the product.

### BRIEF DESCRIPTION OF THE FIGURES

The invention is described in greater detail below with reference to the exemplary embodiments shown in the drawings.
- Figure 1: shows an unused sanitary towel according to the invention, seen from the upper side.
- Figure 2: shows a longitudinal section through the sanitary towel in Figure 1, seen from a long side.
- Figure 3a: shows a cross section through the sanitary towel in Figure 1, seen from a short side.
- Figure 3b: shows a detail from Figure 3a.
- Figure 3c: shows a cross section through an unused sanitary towel according to a second embodiment of the invention.
- Figure 4: shows a used sanitary towel according to Figures 1-3.
- Figure 5: shows a sanitary towel according to a third embodiment of the invention.
- Figure 6: shows a section along the line VI-VI through the sanitary towel in Figure 5 in the unused state.
- Figure 7: shows a section along the line VI-VI through the sanitary towel in Figure 5 in the used state.
- Figure 8: shows a sanitary towel according to a fourth embodiment of the invention, seen from above.
- Figure 9: shows a section along the line IX-IX through the sanitary towel in Figure 8 in the unused state.
- Figure 10: shows a section along the line IX-IX through the sanitary towel in Figure 8 in the used state.
- Figure 11: shows a sanitary towel according to a fifth embodiment of the invention, seen from above.
- Figure 12: shows a section along the line XII-XII through the sanitary towel in Figure 11 in the unused state.
- Figure 13: shows a section along the line XII-XII through the sanitary towel in Figure 11 in the used state.
- Figure 14: shows the production of a raised portion intended for the third, fourth and fifth embodiments of the invention.
- Figure 15: shows the production of a raised portion intended for the third and fifth embodiments of the invention.

### DETAILED DESCRIPTION OF THE FIGURES AND EMBODIMENTS

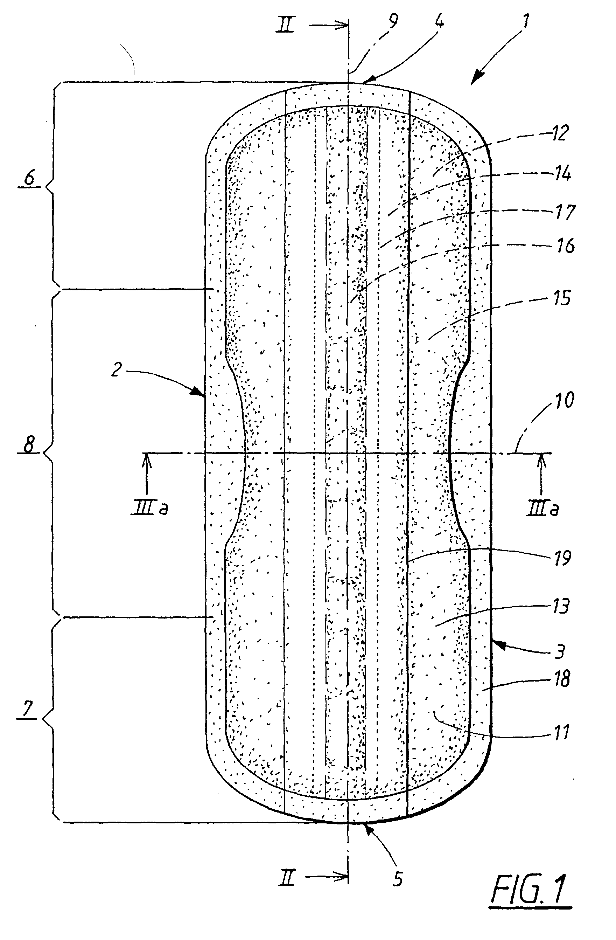

Figures 1-3a show an unused sanitary towel 1 according to one embodiment of the invention.

The sanitary towel 1 has an essentially elongate shape with a longitudinal direction and a transverse direction and has two long sides 2, 3, two short sides 4, 5, two end portions 6, 7 and a central portion 8 located between the end portions, and also a longitudinal centre line 9 extending in the longitudinal direction of the sanitary towel, as well as a transverse centre line 10 extending in the transverse direction of the sanitary towel. A longitudinal centre line means a line extending in the longitudinal direction of the sanitary towel and located equidistantly from the long sides 2, 3 of the sanitary towel. A transverse centre line means a line located in the transverse direction of the sanitary towel equidistantly from the short sides 4, 5 of the sanitary towel. The sanitary towel 1 has an upper side 11 intended to face towards the wearer during use and a lower side 12 intended to face away from the wearer during use.

The sanitary towel 1 comprises a liquid-permeable surface layer 13 arranged on that side of the sanitary towel 1 which is intended to face towards the wearer during use, the upper side 11, and a liquid barrier rear side layer 14 arranged on that side of the sanitary towel which is intended to face away from the wearer during use, the lower side 12. An absorption body 15 is arranged between the surface layer 13 and the liquid barrier rear side layer 14. Part of the absorption body 15 is arranged as a raised portion 16. The raised portion 16 is arranged centrally in the longitudinal direction and transverse direction of the sanitary towel in the central portion 8 of the sanitary towel. The raised portion 16 has a width at the base of roughly 5-10 mm and a height, calculated as the difference from other parts of the absorption body 15, which is roughly 5 mm. It is of course possible for the raised portion 16 to have different heights depending on what is desired in the case in question. Suitable dimensions for raised portions are described in International Patent Application no. PCT/SE97/01886. The dimensions of the raised portion 16 according to the present invention differ from the raised portion described in PCT/SE97/01886 in that this publication describes ready-shaped raised portions, while a raised portion 16 according to the present invention has a first height before use and a second height when the raised portion has been activated and swollen during use.

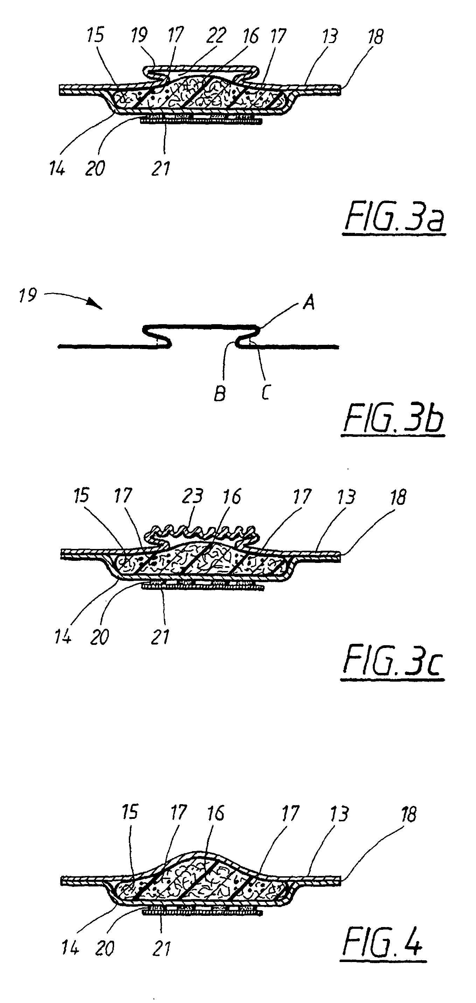

The absorption material forming the raised portion 16 is more compressed than the surrounding parts of the absorption body 15. This has been brought about by partly compressing the absorption body 15. In this embodiment, the raised portion 16 and the absorption body 15 constitute a coherent unit As a result of this construction, the raised portion 16 can swell on wetting. In order for the swelling to be controlled, the attachment areas 17 are in the form of welded spots arranged between the surface layer 13 and the absorption body 15. The attachment areas 17 can of course take a form other than spots. The liquid-permeable surface layer 13 and the liquid barrier layer 14 are joined together outside the edge of the absorption body in a joint 18. The joint 18 can, in a conventional manner, be an adhesive joint or a welded joint. The liquid-permeable surface layer 13 is laid in a fold 19 over the central part of the sanitary towel which has the raised portion 16. The fold 19 is intended to provide space for swelling. Figure 3b shows the fold 19 in Figure 3a in detail.

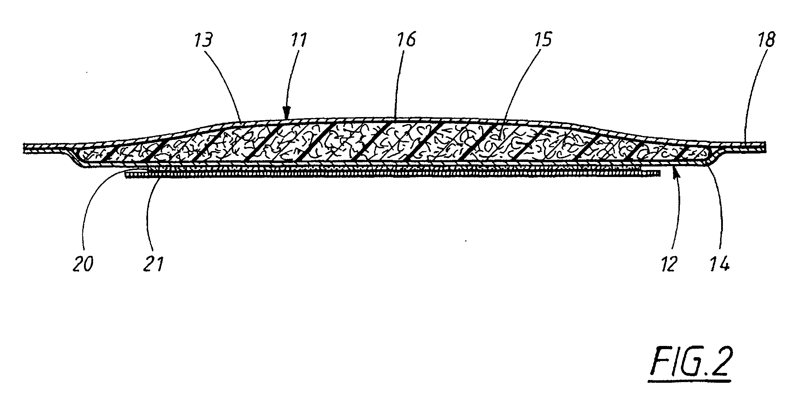

Figure 2 shows a section through the sanitary towel 1 in Figure 1 along the line II-II. On the lower side 12 of the sanitary towel, on its liquid barrier layer 14, a fastening member in the form of lines of pressure-sensitive adhesive 20 is arranged parallel to the longitudinal centre line 9 of the sanitary towel. A detachable protective layer 21 is arranged over the adhesive 20. The protective layer 21 is removed by the wearer before fitting in the undergarment of the wearer. Other fastening members, such as hook-and-loop fasteners or friction fastening, are of course also possible. If the sanitary towel 1 is shaped physically in a special manner and is sufficiently rigid, fastening members can be omitted. Such shaping of products is described in International Patent Application no. PCT/SE97/01882.

Figure 3a shows a cross section through the sanitary towel in Figure 1 along the line IIIa-IIIa. It can be seen in the figure that a cavity 22 is arranged between the surface layer 13 and the absorption body 15 in the area for the raised portion 16.

Figure 3b shows how the possibilities the raised portion 16 has for swelling can be varied by changing the distances between the points A, B and C. A is the outermost edge on the fold, B is the innermost edge on the fold and C is the point of the joining together of the surface layer 13 and the absorption body 15 in the area of the fold 19. For example, if the distance between A and C is increased, greater possibility for the raised portion 16 to swell is obtained.

Figure 3c shows an alternative embodiment of the invention. The only difference from the embodiment described above and shown in Figures 1-3b is that Figure 3c shows a sanitary towel on which an excess of surface material is arranged as wrinkles 23 on top of the absorption body 15.

Figure 4 shows a cross section through a sanitary towel according to the embodiments in Figures 1-3 when it has been used, that is to say the raised portion 16 has swollen and filled the entire excess volume of the surface layer. Irrespective of whether the surface layer has been laid in a fold or wrinkles, the sanitary towel 1 looks the same after swelling. After controlled swelling, the raised portion 16 has a height of roughly 10-20 mm, preferably roughly 13 mm.

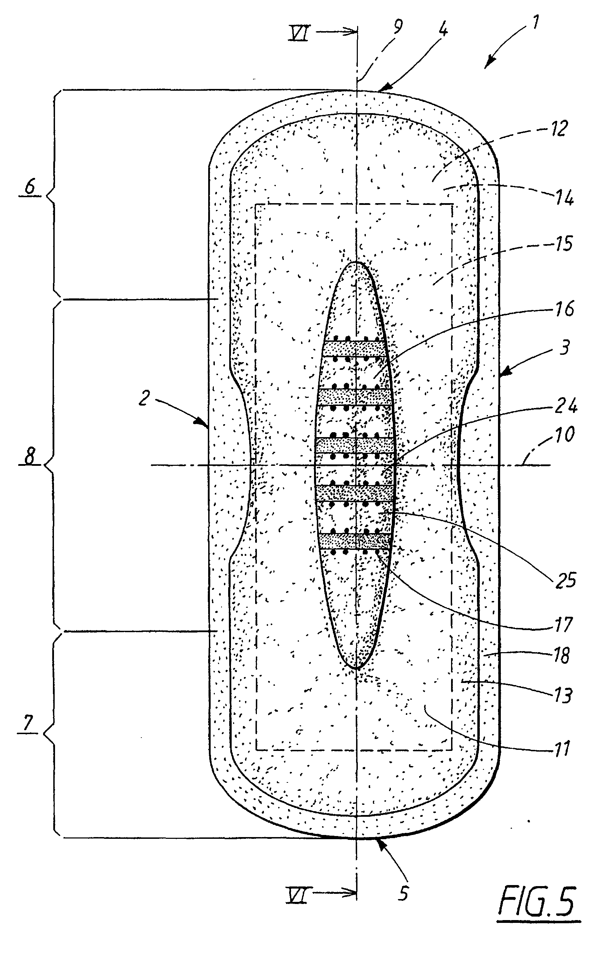

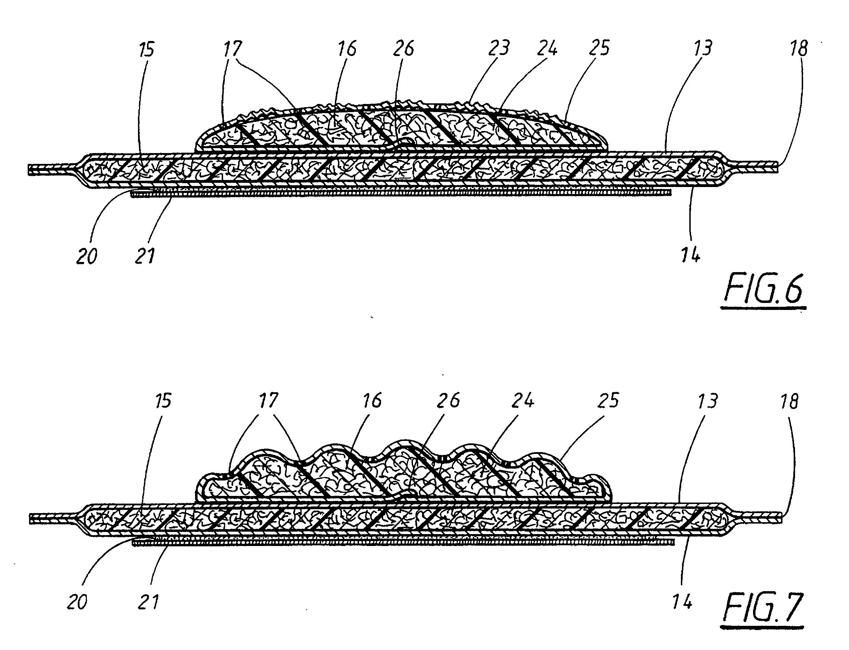

Figures 5-7 show a third embodiment of the invention. Figure 5 shows a sanitary towel 1 seen from the upper side 11, Figure 6 shows a longitudinal section along the line VI-VI through the sanitary towel 1 in Figure 5 before use and Figure 7 shows a longitudinal section through the sanitary towel 1 in Figure 5 when it has been used, that is to say wetted. Seen from above, the sanitary towel 1 looks much the same before and after wetting because the raised portion 16 swells mostly in terms of height, so only one figure, Figure 5, shows the sanitary towel seen from above, and therefore Figure 5 is common to both the unused and the used product.

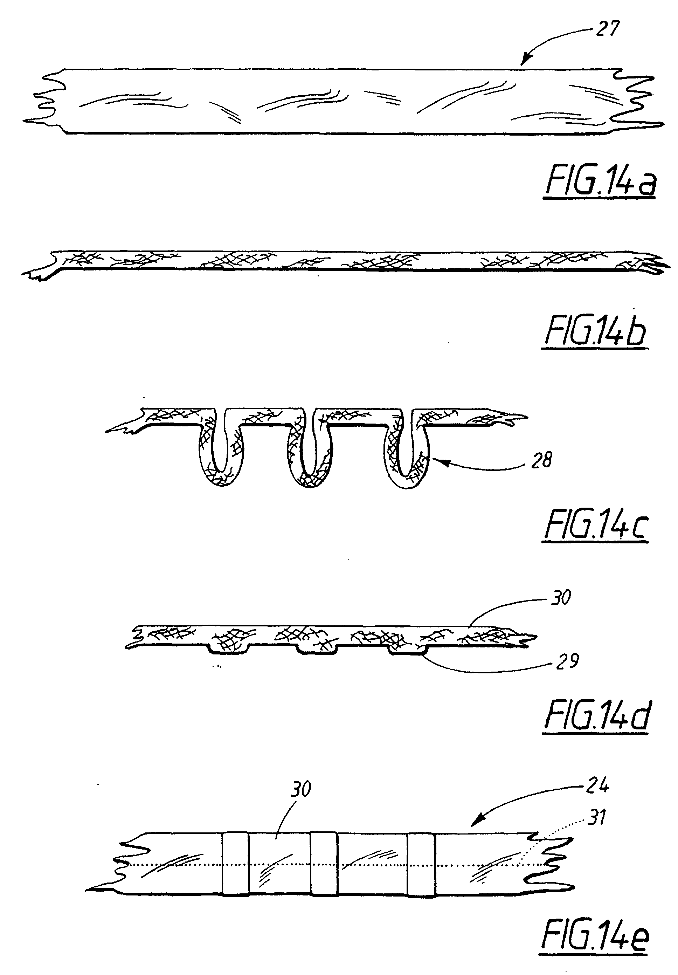

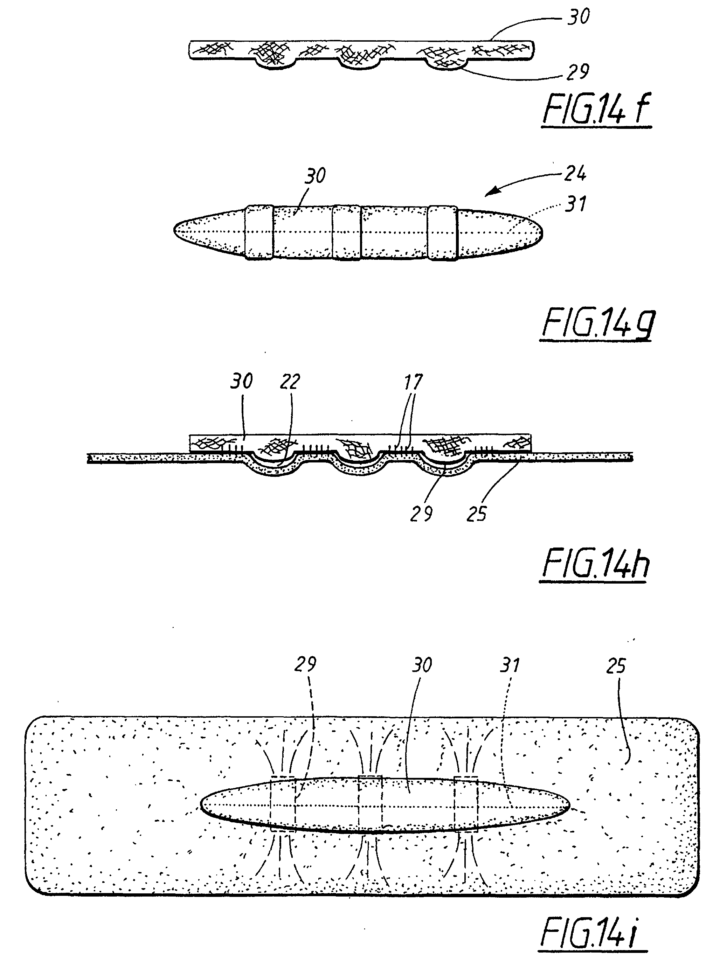

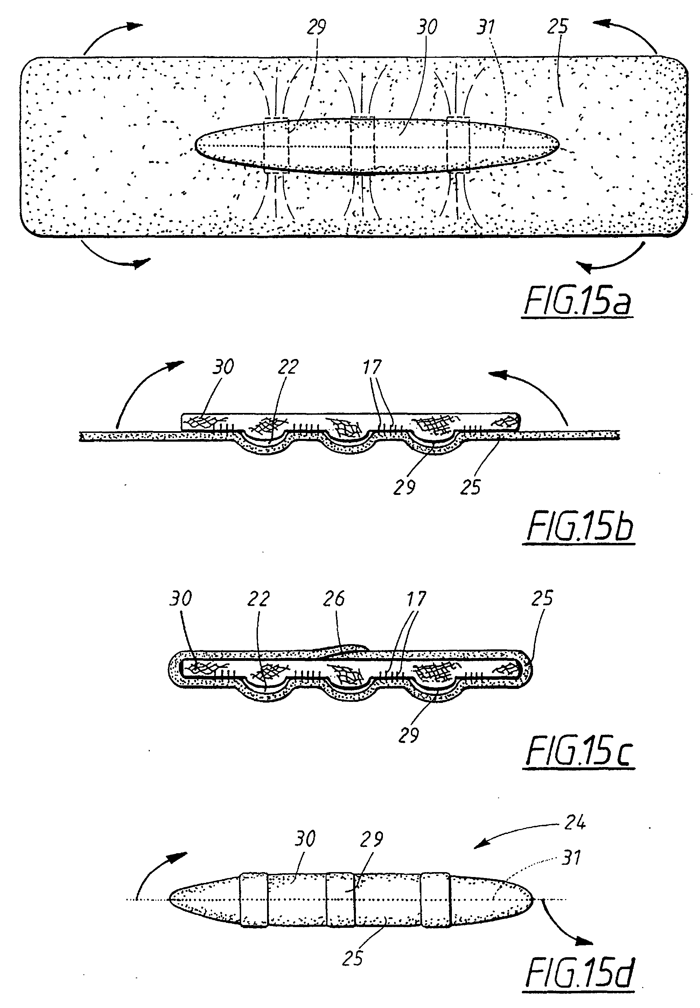

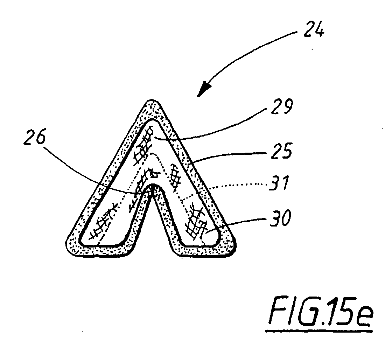

Figures 5-7 shows a sanitary towel 1 which comprises a liquid-permeable first surface layer 13 arranged on that side of the sanitary towel which is intended to face towards the wearer during use and a liquid barrier layer 14 arranged on that side of the sanitary towel which is intended to face away from the wearer during use. A first absorption body 15 is arranged between the first surface layer 13 and the liquid barrier rear side layer 14. The first surface layer 13 and the first absorption body 15 are joined together by means of adhesive over the entire surfaces. A raised portion 16 is arranged on top of the first surface layer 13. The raised portion 16 comprises a second absorption body 24 and a second surface layer 25. The second absorption body 24 is wrapped in the second surface layer 25. The second surface layer 25 is joined together in a joint 26 on the lower side of the raised portion 16. The second surface layer 25 is fastened to the second absorption body 24 by means of fastening points 17. The fastening points 17 consist of binding fibres which are melted by means of supplying heat. The joining together of the second absorption body 24 and the second surface layer 25 is described in greater detail in connection with Figure 15. The excess of surface material to allow swelling of the raised portion 16 is arranged in the form of wrinkles 23 in the second surface layer 25. The fastening points 17 are arranged in such a manner that the second absorption body 24 can swell in a controlled manner and fill the excess of the second surface layer 25. The raised portion 16 is arranged centrally in the longitudinal direction and transverse direction of the sanitary towel. The raised portion 16 has essentially the same dimensions as described above in connection with Figures 1-3. In this embodiment, the raised portion 16 comprising the second absorption body and the first absorption body 15 consist of separate parts. The second absorption body 24 has been made using a method in which the absorption material was shaped with raised sections which were then compressed so that the second absorption body was given uniform thickness but varying density. When the raised portion 16 is wetted and absorbs fluid, the compressed raised sections rise again and impart a corrugated appearance as shown in Figures 7, 10 and 13 to the raised portion 16. Suitable material for the second absorption body 24 is cellulose fluff pulp with high critical bulk, for example chemothermomechanical cellulose fluff pulp (CTMP) or carded rayon fibre. In order for the swelling to be controlled, the fastening areas 17 are in the form of welded spots arranged between the second surface layer 25 and the second absorption body 24. The fastening areas 17 can of course take a form other than spots. The method of producing the raised portion 16 is described in greater detail in connection with Figure 14. The first surface layer 13 and the liquid barrier layer 14 are joined together outside the edge of the absorption body in a joint 18. The joint 18 can be an adhesive joint or a welded joint.

The second surface layer 25 is preferably a nonwoven but can also be a perforated plastic film or a laminate consisting of a perforated plastic film and a nonwoven. The crucial factor for the choice of material is that the material is only slightly stretchable. A surface layer with considerable stretchability would disrupt the controlled swelling in the embodiments comprising folded or wrinkled surface layers. The nonwoven material can be made of, for example, rayon or cotton fibre with a binding fibre, such as polyvinyl alcohol, mixed in. Examples of suitable materials for embodiments with a folded or wrinkled surface layer are Novelin 380-18 made by Suominen or Thermix ES-C made by Tenotex Spa. These materials are nonwovens made of bicomponent fibres made of polyethylene and polypropylene.

The first surface layer 13 can be made of any conventional material, for example nonwoven, perforated plastic film or a laminate consisting of a perforated plastic film and a nonwoven.

The first absorption body 15 is suitably made of cellulose pulp. This may originally be in the form of rolls, bales or sheets which, in the production of the sanitary towel, are dry-defibrated and converted in fluffed form into a pulp mat, sometimes with the addition of so-called superabsorbents which are polymers with the capacity to absorb several times their own weight of water or bodily fluid. An alternative to this is to dry-shape a pulp mat as described in WO 94/10956. Examples of other absorption materials which can be used are various types of natural fibres such as cotton fibres, peat or the like. It is of course also possible to use absorbent synthetic fibres, or mixtures of natural fibres and synthetic fibres. The absorption body 15 can also contain other components, such as dimensionally stabilizing means, liquid-dispersing means, or binders such as, for example, thermoplastic fibres which have been heat-treated in order to hold short fibres and particles together as a coherent unit. It is also possible to use different types of absorbent foam material in the absorption body.

Figure 7 shows a longitudinal section through a sanitary towel according to Figure 5 which has been allowed to swell. The swelling is controlled by a combination of fastening points 17 and an excess of surface material which thus together constitute means for controlled swelling. The second absorption body 24 has now filled the material excess in the second surface layer 25. The second absorption body 24 and the raised portion 16 have a corrugated surface after swelling.

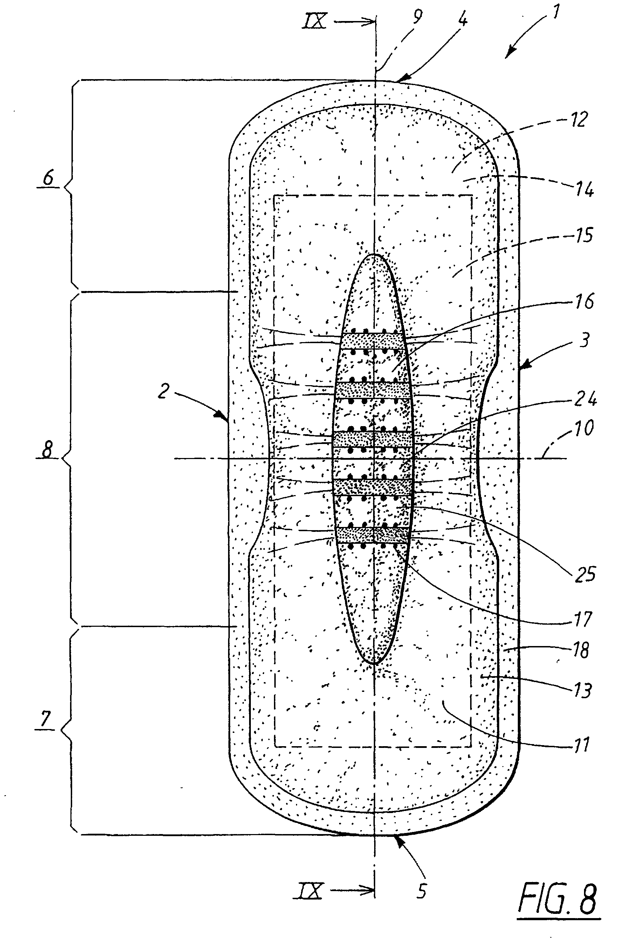

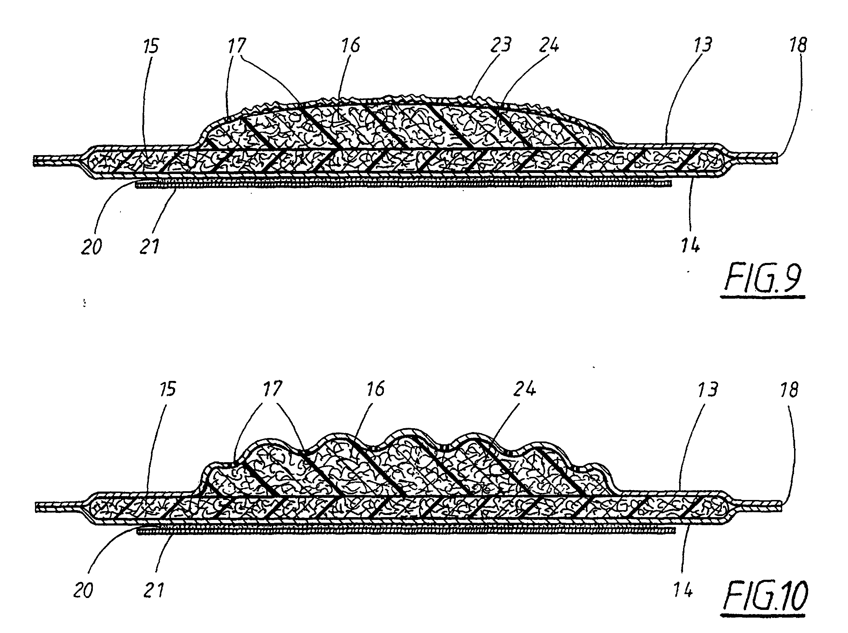

Figures 8-10 show a sanitary towel 1 according to a fourth embodiment of the invention. The embodiment is a variant of the third embodiment, the difference being that the surface layers 13, 25 have been arranged in a different manner. Figure 8 shows the sanitary towel 1 seen from its upper side 11, Figure 9 shows a longitudinal section through the sanitary towel 1 in the dry state and Figure 10 shows a longitudinal section through the sanitary towel 1 after wetting. In Figures 9 and 10, it can be seen that the surface layer 13 is common to the first absorption body 15 and the second absorption body 24. The surface layer 13 is fastened to the raised portion by fastening points 17 and fastened to the rear side layer 14 in an edge joint 18 located outside the first absorption body 15.

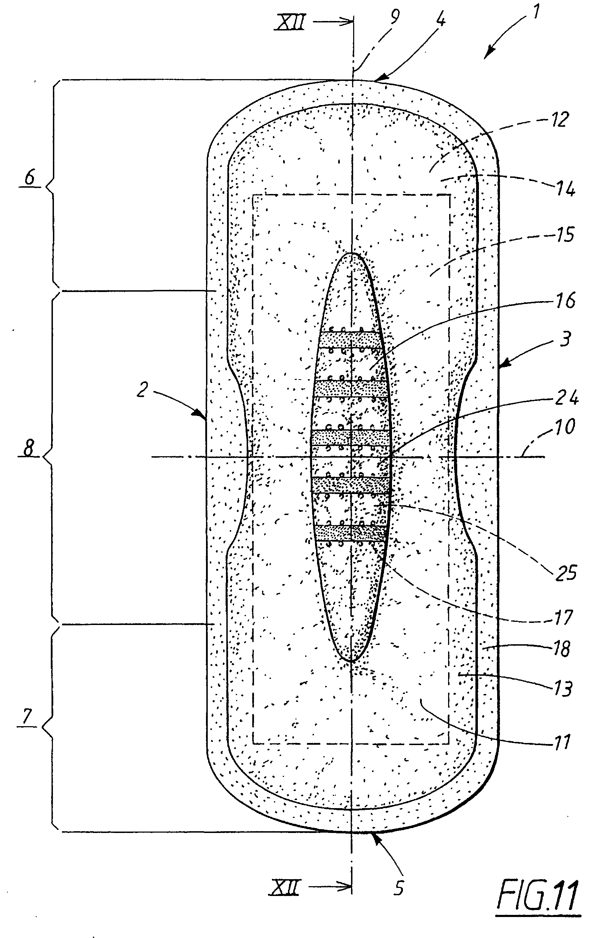

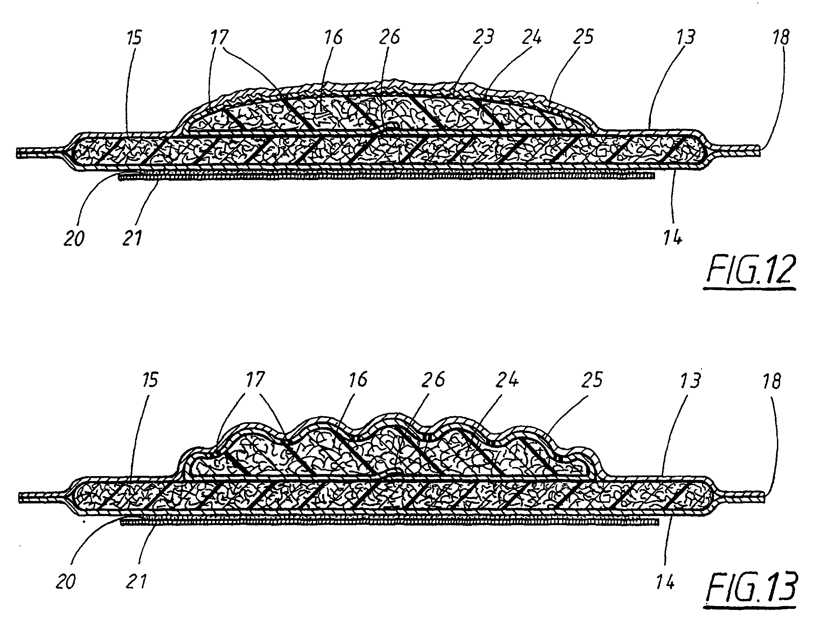

Figures 11-13 show a sanitary towel 1 according to a fifth embodiment of the invention. The embodiment is another variant of the third embodiment, the difference being that the surface layer has been arranged in a different manner again. Figure 11 shows the sanitary towel 1 seen from above, Figure 12 shows a longitudinal section through the sanitary towel 1 in the dry state and Figure 13 shows a longitudinal section through the sanitary towel 1 after wetting. Figures 12 and 13 show how a first surface layer 13 is laid over both the first absorption body 15 and the second absorption body 24. The first surface layer 13 is fastened to the rear side layer 14 in an edge joint 18 located outside the first absorption body 15. A second surface layer 25 encloses, and is fastened at fastening points 17 to, the second absorption body 24. The first surface layer 13 covers the second surface layer 25. The joining together of the second absorption body 24 and the second surface layer 25 is described in greater detail in connection with Figure 15.

Figure 14 shows the production of a compressed raised portion 16 according to the invention. Figure 14a shows a carded fibre strip 27 made of rayon, seen from above. The strip can of course be made of another material, for example of the types described above, in connection with Figures 5-13, as being suitable for the second absorption body 24. Figure 14b shows the fibre strip 27 seen from the side. Figure 14c shows the fibre strip 27 when it has been shaped into loops 28 in a mould. Figure 14d shows the fibre strip 27 when it has been compressed in the mould so that areas 29 with a greater degree of compression have been created in the fibre strip 27. These areas 29 have greater density and a greater swelling possibility on wetting than surrounding areas 30 of low compression. A longitudinal creasing line 31 is made in the fibre strip 27 simultaneously with the compression. Figure 14e shows the fibre strip 27 seen from above in the compressed state. Figure 14f shows the second absorption body 24 seen from the side, when it has been punched out from the partly compressed fibre strip 27 in a shaping punch. Figure 14g shows the second absorption body 24, seen from above, when it has been punched out.

Figure 14h shows how, after punching out, the second absorption body 24 is laid down on a surface layer 25. The surface layer 25, which has been referred to above as the second surface layer, is a nonwoven made of fibres with different melting points. The fibre with the lower melting point then constitutes what is known as a binding fibre. The surface layer 25 is laid over a mould which provides a predetermined excess of material. The second absorption body 24 is pressed down into the mould and heated. By applying pressure in areas by means of a tool, attachment areas 17 are obtained when heat is supplied, within which areas the binding fibre melts, the second surface layer 25 and the second absorption body 24 then being fastened together. A means for controlled swelling of the raised portion has thus been brought about. The attachment areas 17 can take the form of, for example, spots or lines. The attachment areas 17 are to have sufficient strength to be capable of holding the surface layer 25 and the second absorption body 24 together when the absorption material swells. The attachment areas 17 are arranged in the areas 30 of low compression on the second absorption body 24, leaving the areas 29 of high compression free to swell and fill the excesses of surface material and the cavities 22 located above the areas of high compression. The attachment areas 17 are arranged in the transition between areas of low compression and areas of high compression. This means that on the whole the absorption material only swells upwards and not to the sides. Another desirable effect of this positioning of the fastening areas is that the surface layer follows the absorption material when it swells and is not stretched so that an air pocket is formed between the surface layer 25 and the absorption body 24. Such an air pocket would render further liquid transfer more difficult. Figure 14i shows the raised portion seen from above.

Figures 15a-e show the folding over of the second surface layer 25 around the second absorption body 24. Figure 15a shows diagrammatically how the second surface layer 25 is folded in around the second absorption body 24. Figure 15b shows the same operation as in Figure 15a, but seen from the side. Figure 15c shows a side view in which the second surface layer 25 has been folded around the second absorption body 24. Figure 15d shows how the second absorption body 24 surrounded by the second surface layer 25 is folded along the creasing line 31 in order to create the raised portion 16. Figure 15e shows the raised portion 16 seen from one of its short sides after folding. A tool goes up under the raised portion 16 and meets on the opposite side, heat is supplied and the second surface layer 25 is welded together with itself on the lower side of the raised portion in a joint 26.

In the fourth embodiment, shown in Figures 8-10, the surface layer 13 is the same for the first absorption body 15 and the second absorption body 24. After the stages shown in Figure 14, the surface layer 13 is joined to the rear side layer 14 in an edge joint 18 by means of adhesive or heat.

The liquid barrier layer 14, or the rear side layer, consists of a liquid-impermeable material. Thin, liquid-tight plastic films are suitable for the purpose but it is also possible to use material which is originally liquid-permeable but has been provided with a coating of plastic, resin or another liquid-tight material. In this way, leakage of fluid from the lower side of the absorbent product is prevented. The barrier layer 14 can thus consist of any material that satisfies the criterion of liquid-impermeability and is also sufficiently flexible and skin-friendly for the purpose. Examples of suitable materials for the barrier layer are plastic films, nonwovens and laminates consisting of these. The plastic film may be made of, for example, polyethylene, polypropylene or polyester. Alternatively, the barrier layer can consist of a laminate made of a liquid-impermeable plastic film facing towards the absorption body and a nonwoven facing towards the undergarment of the wearer. Such a construction provides a leakproof barrier layer with a textile feel.

The invention is not to be considered as limited to the embodiments above. These are intended only to explain the invention.

It is possible to combine characteristics from different embodiments with one another within the scope of the invention. For example, all the methods described of creating the excess of surface material can be combined with different embodiments of raised portions.

Controlled swelling of the raised portion can also be permitted by virtue of the surface layer being stretchable and fastening points being arranged in suitable places between surface layers and absorption bodies. Surface layers can be stretchable by virtue of inherent properties in the material or how the surface layer has been made physically, for example knitted or woven. The stretching that occurs on wetting must in this case be known, so that uncontrolled stretching does not take place. If a central raised portion is to be created, it is also important that the stretching is not too great, so that the raised portion becomes uncomfortable for the wearer. Stretchable surface layers suitable for this embodiment are, for example, cotton stockinet, perforated plastic film or a laminate consisting of a perforated plastic film and a nonwoven layer. A commercially available material suitable for this application is Fibrella 2000 made by Suominen. Fibrella 2000 is a spun-lace nonwoven made of rayon fibre.

## Claims

1. Absorbent product, such as a sanitary towel, an incontinence pad or a panty liner, which product has an essentially elongate shape with a longitudinal direction and a transverse direction, an upper side (11) and a lower side (12), and has two end portions (6, 7) and a central portion (8) located between the end portions, the upper side (11) of which product has in the central portion (8) a raised portion (16) comprising an absorbent material which can absorb on wetting, and a liquid-permeable surface layer (13;25) arranged over the raised portion (16), wherein the product comprises means for controlled swelling of the raised portion (16) on wetting, **characterized in that** said means for controlled swelling comprises fastening points (17) made of water-soluble material between the liquid-permeable surface layer (13; 25) and the raised portion (16).

2. Absorbent product according to Claim 1, **characterized in that** the raised portion (16) consists of a separate piece of material.

3. Absorbent product according to Claim 2, **characterized in that** a liquid-permeable surface layer (13) is laid over the upper side of the entire product

4. Absorbent product according to Claim 2, **characterized in that** a liquid-permeable surface layer (25) encloses the raised portion (16) and **in that** another surface layer (13) is laid over other parts of the upper side (11) of the product.

5. Absorbent product according to any one of Claims 2, 3 or 4, **characterized in that** the means for controlled swelling comprises fastening points (17) between surface layers (13; 25) and the raised portion (16).

6. Absorbent product according to Claim 5, **characterized in that** the means for controlled swelling comprises a stretchable surface layer (13; 25).

7. Absorbent product according to Claim 5, **characterized in that** the means for controlled swelling comprises an excess (19; 23) of surface layer material.

8. Absorbent product according to any one of the preceding claims, **characterized in that** the water-soluble material is polyvinyl alcohol.

9. Method of producing an absorption structure for the formation of a raised portion in an absorbent product in accordance with claim 1, comprising the following steps:
a) a compressible strip (27) of absorption material is laid down in such a shape that loops (28) of excess material are formed in various areas of the strip (27).
b) the strip (27) is compressed so that the loops (28) are essentially obliterated and areas (29) with a greater degree of compression alternating with areas (30) with a smaller degree of compression are formed.
c) a long, narrow body (24) is punched out of the compressed strip (27),
d) the body (24) is arranged on a surface layer (25) arranged in a mould with depressions,
e) the surface layer (25) is attached discontinuously with a water-soluble material to the absorption body so that an excess of surface layer is obtained between the attachment areas (17).

## Patentansprüche

1. Absorptionsprodukt, wie z.B. Hygienebinde, Inkontinenzpolster oder Slipeinlage, welches Produkt eine im wesentlichen längliche Form mit einer Längsrichtung und einer Querrichtung, eine Oberseite (11) und eine Unterseite (12) sowie zwei Endabschnitte (6, 7) und einen mittleren Abschnitt (8) aufweist, der zwischen den Endabschnitten angeordnet ist, wobei die Oberseite (11) des Produktes in den mittleren Abschnitt (8) einen angehobenen Abschnitt (16) mit einem Absorptionsmaterial, das nach dem Nässen absorbieren kann, und eine flüssigkeitsdurchlässige Oberflächenschicht (13; 25) aufweist, die über den angehobenen Abschnitt (16) angeordnet ist, wobei das Produkt eine Einrichtung für ein gesteuertes Schwellen des angehobenen Abschnittes (16) nach dem Nässen aufweist, **dadurch gekennzeichnet, dass** die Einrichtung für ein gesteuertes Schwellen Befestigungsstellen (17) aufweist, die aus wasserlöslichem Material zwischen der flüssigkeitsdurchlässigen Oberflächenschicht (13; 25) und dem angehobenen Abschnitt (16) ausgebildet sind.

2. Absorptionsprodukt nach Anspruch 1, **dadurch gekennzeichnet, dass** der angehobene Abschnitt (16) aus einem getrennten Materialstrick besteht.

3. Absorptionsprodukt nach Anspruch 2, **dadurch gekennzeichnet, dass** die flüssigkeitsdurchlässige Oberflächenschicht (13) über die Oberseite des gesamten Produktes gelegt ist.

4. Absorptionsprodukt nach Anspruch 2, **dadurch gekennzeichnet, dass** eine flüssigkeitsdurchlässige Oberflächenschicht (25) den angehobenen Abschnitt (16) einschließt, und dadurch, dass eine weitere Oberflächenschicht (13) über andere Teile der Oberseite (11) des Produktes gelegt ist.

5. Absorptionsprodukt nach einem der Ansprüche 2, 3 oder 4, **dadurch gekennzeichnet, dass** die Einrichtung für ein gesteuertes Schwellen Befestigungsstellen (17) zwischen Oberflächenschichten (13; 25) und den angehobenen Abschnitt (16) aufweist.

6. Absorptionsprodukt nach Anspruch 5, **dadurch gekennzeichnet, dass** die Einrichtung für ein gesteuertes Schwellen eine dehnbare Oberflächenschicht (13; 25) aufweist.

7. Absorptionsprodukt nach Anspruch 5, **dadurch gekennzeichnet, dass** die Einrichtung für ein gesteuertes Schwellen einen Überschuss (19; 23) von Oberflächenschichtmaterial aufweist.

8. Absorptionsprodukt nach einem der vorangehenden Ansprüche, **dadurch gekennzeichnet, dass** das wasserlösliche Material Polyvinylalkohol ist.

9. Verfahren zur Herstellung einer Absorptionsstruktur für die Ausbildung eines angehobenen Abschnittes in einem Absorptionsprodukt gemäß Anspruch 1, mit den folgenden Schritten:
a) ein komprimierbarer Streifen (27) aus Absorptionsmaterial wird in einer derartigen Form abgelegt, dass Schlaufen (28) aus überschüssigem Material in verschiedenen Bereichen des Streifens (27) ausgebildet werden,
b) der Streifen (27) wird komprimiert, so dass die Schlaufen (28) im wesentlichen vernichtet werden, und Bereiche (29) mit einem höheren Ausmaß der Kompression, die sich mit Bereichen (30) mit einem geringeren Ausmaß der Kompression abwechseln, ausgebildet werden,
c) ein langer, schmaler Körper (24) wird aus dem komprimierten Streifen (27) gestanzt,
d) der Körper (24) wird an einer Oberflächenschicht (25) angeordnet, die in einer Form mit Vertiefungen angeordnet ist,
e) die Oberflächenschicht (25) wird diskontinuierlich mit einem wasserlöslichen Material an den Absorptionskörper angebracht, so dass ein Überschuss von Oberflächenschicht zwischen den Anbringbereichen (17) erhalten wird.

## Revendications

1. Produit absorbant, tel qu'une serviette hygiénique, une protection contre l'incontinence ou un protège-slip, lequel produit a une forme essentiellement allongée avec une direction longitudinale et une direction transversale, une face supérieure (11) et une face inférieure (12), et comporte deux parties d'extrémité (6, 7) et une partie centrale (8) située entre les parties d'extrémité, la face supérieure (11) comportant dans la partie centrale (8) une partie dressée (16) comprenant un matériau absorbant qui peut absorber lors d'un mouillage, et une couche de surface (13 ; 25) perméable aux liquides placée sur la partie dressée (16), le produit comprenant un moyen pour le gonflement contrôlé de la partie dressée (16) lors du mouillage, **caractérisé en ce que** ledit moyen pour le gonflement contrôlé comprend des points de fixation (17) faits d'un matériau soluble dans l'eau entre la couche de surface (13 ; 25) perméable aux liquides et la partie dressée (16).

2. Produit absorbant selon la revendication 1, **caractérisé en ce que** la partie dressée (16) est constituée d'un morceau de matériau séparé.

3. Produit absorbant selon la revendication 2, **caractérisé en ce qu'**une couche de surface (13) perméable aux liquides est déposée sur la face supérieure du produit entier.

4. Produit absorbant selon la revendication 2, **caractérisé en ce qu'**une couche de surface (25) perméable aux liquides renferme la partie dressée (16) et **en ce qu'**une autre couche de surface (13) est déposée sur les autres parties de la face supérieure (11) du produit.

5. Produit absorbant selon l'une quelconque des revendications 2, 3 ou 4, **caractérisé en ce que** le moyen pour le gonflement contrôlé comprend des points de fixation (17) entre les couches de surface (13 ; 25) et la partie dressée (16).

6. Produit absorbant selon la revendication 5, **caractérisé en ce que** le moyen pour le gonflement contrôlé comprend une couche de surface extensible (13 ; 25).

7. Produit absorbant selon la revendication 5, **caractérisé en ce que** le moyen pour le gonflement contrôlé comprend un excès (19 ; 23) de matériau de couche de surface.

8. Produit absorbant selon l'une quelconque des revendications précédentes, **caractérisé en ce que** le matériau soluble dans l'eau est du poly(alcool vinylique).

9. Procédé de fabrication d'une structure absorbante pour la formation d'une partie dressée dans un produit absorbant conforme à la revendication 1, comprenant les étapes consistant à :
a) déposer une bande compressible (27) de matériau absorbant avec une forme telle que des boucles (28) de matériau en excès sont formées dans diverses zones de la bande (27),
b) comprimer la bande (27) de sorte que les boucles (28) sont essentiellement oblitérées et des zones (29) avec un grand degré de compression alternant avec des zones (30) à petit degré de compression sont formées,
c) découper un corps long et étroit (24) dans la bande comprimée (27),
d) placer le corps (24) sur une couche de surface (25) placée dans un moule muni de dépressions,
e) attacher de manière discontinue la couche de surface (25) avec un matériau soluble dans l'eau sur le corps absorbant de telle manière qu'un excès de couche de surface est obtenu entre les zones de fixation (17).
